# EUROPÄISCHE PATENTSCHRIFT

(11) **EP 1 595 875 B1**
(45) Veröffentlichungstag und Bekanntmachung des Hinweises auf die Patenterteilung: **22.06.2011**
(21) Anmeldenummer: 05009299.8
(22) Anmeldetag: 28.04.2005
(51) Int. Cl.: C07D 317/46

(54) **Verfahren zur Herstellung von Isothiocyanato-2,2-Difluorbenzo-(1,3)-Dioxolen**
Process for the preparation of isothiocyanato-2,2-difluoro-1,3-benzodioxoles
Procédé de préparation d' isothiocyanato-2,2-difluoro-1,3-benzodioxoles

(30) Priorität: 14.05.2004 DE 102004024011
(43) Veröffentlichungstag der Anmeldung: 16.11.2005
(73) Patentinhaber: Saltigo GmbH, 40764 Langenfeld (DE)
(72) Erfinder: Pleschke, Axel Dr., 51069 Köln (DE); Marhold, Albrecht Dr., 51373 Leverkusen (DE)
(74) Vertreter: Wichmann, Birgid

(56) Entgegenhaltungen:
- EP-A- 0 042 533
- WO-A-02/081453
- DE-A1- 2 848 531
- DE-A1- 4 133 156
- US-A- 5 663 189

## Beschreibung

Die Erfindung betrifft ein Verfahren zur Herstellung von Difluorbenzo-[1,3]-dioxolen.

Difluorbenzo-[1,3]-dioxole sind wertvolle Bausteine in agrochemischen und pharmazeutischen Wirkstoffen, siehe auch WO 02/81453, insbesondere Beispiel 321 oder EP 42533. Um den Difluorbenzo-[1,3]-dioxol-Baustein in das Wirkstoffnolekül einführen zu können, wird 5-Brom-difluorbenzo-[1,3]-dioxol einer palladium-katalysierten Transformation unterworfen, die aus industrieller Sicht zu kostenintensiv ist. Es bestand daher das Bedürfnis ein einfaches und effizientes Verfahren zur Herstellung von Difluorbenzo-[1,3]-dioxolen bereitzustellen.

Die Verbindungen der Formel (I), 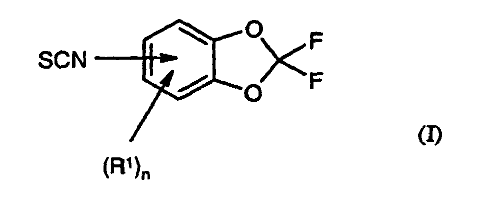 in der
- R¹: für C₁-C₁₂-Alkyl, C₁-C₁₂-Fluoralkyl, C₁-C₁₂-Alkoxy, C₁-C₁₂-Fluoralkoxy, Cyano, Vitro. Iod, Brom, Chlor oder Fluor und
- n: für 0, 1, 2 oder 3 steht
lassen sich in einfacher und aus WO 02/81453 bekannter Weise zu dort beschriebenen Thioxoimidazolinonen umsetzen.

Es wurde ein Verfahren zur Herstellung von Verbindungen der Formel (I) gefunden, das dadurch gekennzeichnet ist, dass Verbindungen der Formel (II) 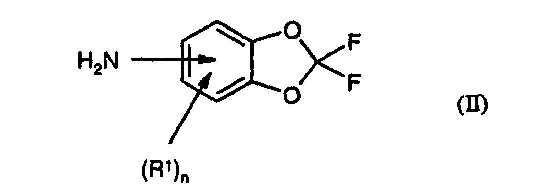 in der R¹ und n die für die Formel (I) angegebene Bedeutung haben, mit Thiophospgen in einem wässrig-sauren Medium oder mit Chlorthiocarbonylimidazol oder mit Thiocarbonylbisimidazol umgesetzt werden.

Die genannten Verfahren sind aus der Literatur vom Prinzip her hinlänglich bekannt. Die als Ausgangsverbindungen verwendeten Verbindungen der Formel (II) sind entweder aus der Literatur bekannt oder analog zur Literatur herstellbar. Als besonders überraschend ist zu werten, dass die bekanntermaßen labile sowie temperatur- und gegen Halogenaustausch empfindliche Difluorbenzodioxol-Funktion durch die Umsetzung nicht in nennenswertem Umfang angegriffen wird.

Der Rahmen der Erfindung umfasst alle im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Alkyl beziehungsweise Alkoxy steht jeweils unabhängig für einen geradkettigen, cyclischen, verzweigten oder unverzweigten Alkyl- beziehungsweise Alkoxy-Rest, wobei die genannten Reste gegebenenfalls durch C₁-C₄-Alkoxy-Reste weiter substituiert sein können.

C₁-C₁₂-Alkyl steht beispielsweise und bevorzugt für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, n-Decyl und n-Dodecyl.

C₁-C₁₂-Alkoxy steht beispielsweise und bevorzugt für Methoxy, Ethoxy, n-Propoxy, iso-Propoxy, n-Butoxy, sec.-Butoxy, tert.-Butoxy, n-Pentoxy, cyclo-Hexoxy, cyclo-Pentoxy, n-Hexoxy, n-Heptoxy, n-Octoxy, n-Decoxy und n-Dodecoxy

**Fluoralkyl** beziehungsweise **Fluoralkoxy** steht jeweils unabhängig für einen geradkettigen, cyclischen, verzweigten oder unverzweigten Alkyl-Rest beziehungsweise Alkoxy-Rest, der einfach, mehrfach oder vollständig durch Fluoratome substituiert ist.

Beispielsweise steht C₁-C₁₂-Fluoralkyl für Trifluormethyl, 2,2,2-Trifluorethyl, Pentafluorethyl, Nonafluorbutyl, Heptafluorisopropyl, Perfluoroctyl und Perfluordodecyl.

Beispielsweise steht C₁-C₁₂-Fluoralkoxy für Trifluormethoxy, 2,2,2-Trifluorethoxy, Pentafluorethoxy, Nonafluorbutoxy, Heptafluorisopropoxy, Perfluoroctoxy und Perfluordodecoxy.

Im Folgenden werden die bevorzugten Substitutionsmuster der Verbindungen der Formeln (I) und (II) definiert:
- R¹: steht besonders bevorzugt für Methyl, Ethyl, n-Propyl, Chlor, Fluor und Brom, ganz besonders bevorzugt für Chlor oder Fluor.
- n: steht bevorzugt für 0, 1 oder 2, besonders bevorzugt für 0 oder 1 und ganz besonders bevorzugt für 0.

Eine besonders bevorzugte Verbindung der Formel (I) ist 5-Isothiocyanato-2,2-difluorbenzo-[1,3]-dioxol.

Eine besonders bevorzugte Verbindung der Formel (II) ist 5-Amino-2,2-difluorbenzo-[1,3]-dioxol.

Die erfindungsgemäßen Verbindungen der Formel (I) eignen sich insbesondere zur Herstellung von Arzneimitteln, Agrochemikalien oder Zwischenprodukten davon. Vorzugsweise können Verbindungen der Formel (I) zur Herstellung von Thioxoimidazolinonen eingesetzt werden, insbesondere für solche, wie sie in WO 02/81453 beschrieben sind.

### Beispiele:

### Beispiel 1:

### Herstellung von 5-Isothiocyanato-2,2-difluorbenzo[1,3]dioxol:

34,6 g 5-Amino-2,2-difluorbenzodioxol werden zu einer Lösung von 90 ml konz. Salzsäure in 350 ml Wasser zugetropft. Es wird für 30 Minuten nachgerührt und dann werden rasch 27 g Thiophosgen andosiert. Es wird bei gegebener Temperatur für 3 Stunden nachgerührt, die fast farblose Suspension mit 150 ml Dichlormethan versetzt und das Dichlormethan abgezogen. Das Rohprodukt wird bei 45 mbar destilliert. Man erhält 28 g (= 89 % d. Th.) als farblose bis gelbliche Flüssigkeit.
Siedepunkt: 136°C / 45 mbar.
¹H-NMR (400 MHz, CDCl₆): 6.96 (m, 2H), 7.02 (m, 1H)

## Patentansprüche

1. Verfahren zur Herstellung von Verbindungen der Formel (I), 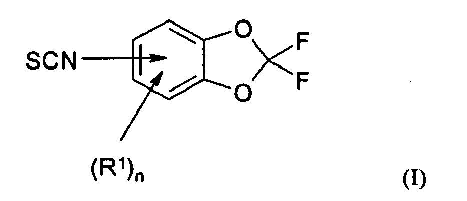 in der
R¹ für C₁-C₁₂-Alkyl, wobei C₁-C₁₂-Alkyl weiter substituiert sein kann durch C₁-C₄- Alkoxy-Reste, C₁-C₁₂-Fluoralkyl, C₁-C₁₂-Alkoxy, wobei C₁-C₁₂-Alkoxy weiter substituiert sein kann durch C₁-C₄-Alkoxy-Reste, C₁-C₁₂-Fluoralkoxy, Cyano, Nitro, Iod, Brom, Chlor oder Fluor und
n für 0, 1, 2 oder 3 steht,
durch Umsetzung von Verbindungen der Formel (II), 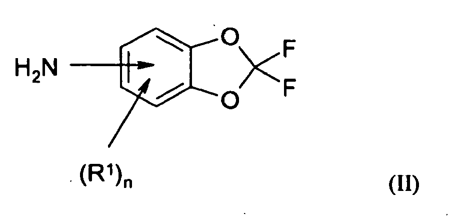 in der R¹ und n die für die Formel (I) angegebene Bedeutung haben,
mit Thiophospgen in einem wässrig-sauren Medium oder mit Chlorthiocarbonylimidazol oder mit Thiocarbonylbisimidazol.

## Claims

1. Process for preparing compounds of the formula (I) 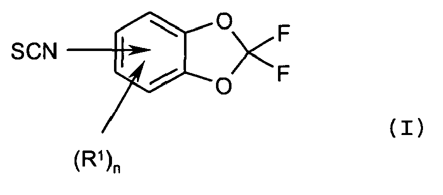 in which
R¹ is C₁-C₁₂-alkyl, where C₁-C₁₂-alkyl may be further substituted by C₁-C₄-alkoxy radicals, C₁-C₁₂-fluoroalkyl, C₁-C₁₂-alkoxy, where C₁-C₁₂- alkoxy may be further substituted by C₁-C₄- alkoxy radicals, C₁-C₁₂-fluoroalkoxy, cyano, nitro, iodine, bromine, chlorine or fluorine and
n is 0, 1, 2 or 3,
by reacting compounds of the formula (II), 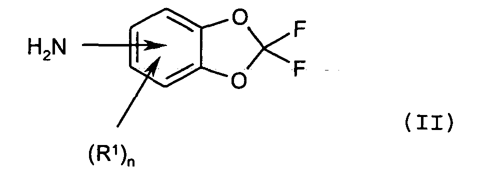 in which R¹ and n are as defined for the formula (I), with thiophosgene in an aqueous-acidic medium or with chlorothiocarbonylimidazole or with thiocarbonylbisimidazole.

## Revendications

1. Procédé de préparation de composés de la formule (I) 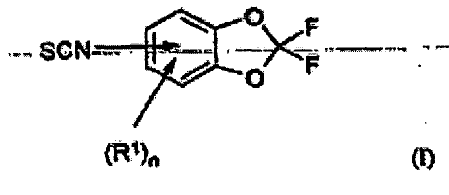 dans laquelle
R¹ représente un groupe alkyle en C₁-C₁₂, dans lequel le groupe alkyle en C₁-C₁₂ peut en outre être substitué par des radicaux alcoxy en C₁-C₄, un groupe fluoroalkyle en C₁-C₁₂, un groupe alcoxy en C₁-C₁₂, dans lequel le groupe alcoxy en C₁-C₁₂ peut en outre être substitué par des radicaux alcoxy en C₁-C₄, un groupe fluoroalcoxy en C₁-C₁₂, un groupe cyano, nitro, iode, brome, chlore ou fluor et
n vaut 0, 1, 2 ou 3,
par conversion de composés de la formule (II) 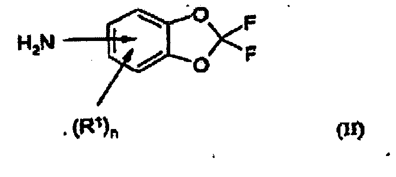 dans laquelle
R¹ et n ont la signification indiquée pour la formule (I)
avec du thiophosgène dans un milieu aqueux acide ou avec du chlore thiocarbonylimidazole ou avec du thiocarbonylbisimidazole.
